# EUROPEAN PATENT APPLICATION

(11) **EP 4 455 670 A1**
(43) Date of publication of application: **30.10.2024**
(21) Application number: 22911832.8
(22) Date of filing: 20.12.2022
(51) Int. Cl.: G01N 33/68, C12Q 1/6883, A61K 31/4245, A61P 43/00

(54) **ACTIVATED RAS AS THERAPEUTIC AND DIAGNOSTIC TARGET FOR NEUROFIBROMATOSIS AND USE THEREOF**

(30) Priority: 21.12.2021 KR 20210183885
(71) Applicant: The Asan Foundation, Seoul 05505 (KR); University Of Ulsan Foundation For Industry Cooperation, Ulsan 44610 (KR); Humanscape Inc., Gangnam-gu Seoul 06163 (KR)
(72) Inventor: LEE, Beom Hee, Seoul 05530 (KR); CHANG, Min Hoo, Seoul 06196 (KR); HEO, Sun Hee, Seoul 05505 (KR); DO, Hyo Sang, Seoul 05333 (KR); KANG, Min Ji, Seoul 05505 (KR)
(74) Representative: AWA Sweden AB
(86) International application number: PCT/KR2022/020770
(87) International publication number: WO 2023/121199

(57) **Abstract**

The present disclosure relates to: a biomarker composition for predicting susceptibility to a therapeutic agent for neurofibromatosis by using Ras activity; and a pharmaceutical composition for preventing or treating neurofibromatosis, including a Ras activity inhibitor. The biomarker composition can accurately diagnose susceptibility to a therapeutic agent for neurofibromatosis through a simple method of measuring the expression levels of a GTP-bound and activated Ras (Ras-GTP) protein and an activated p-ERK protein or a gene encoding the same in a biological sample of an individual, and the pharmaceutical composition inhibits the activity of Ras proteins, and thus can be effectively used for the prevention or treatment of neurofibromatosis in which the activity of the Ras proteins is increased.

## Description

### [Technical Field]

The present disclosure relates to activated Ras as a therapeutic and diagnostic target for neurofibromatosis.

### [Background Art]

Neurofibromatosis type 1 (NF1) is an autosomal dominant genetic disease, and is one of genetic diseases with a relatively high incidence of 1 in 3,000 people worldwide. NF1 is caused by a mutation in the NF1 gene and is characterized by multiple cafe-au-lait-spots and neurofibromas of the skin, and various levels of neurologic, skeletal, and neoplastic expressions can have a significant impact on patients. NF1 patients are at risk of developing tumors in the central and peripheral nervous systems, at a rate 1,000 times higher than normal people.
existing treatment method such as chemotherapy, radiation therapy, surgical resection, and the like is the only existing treatment for NF1, but in cases where NF1 occurs multiple times in multiple organs, surgery is often impossible.

Recently, for use as a non-invasive therapeutic agent for NF1 patients, an MEK inhibitor selumetinib was approved by the FDA and is available as an orphan in Korea. As another therapeutic agent, a tyrosine kinase inhibitor carbozantinib has been reported to be safe and tumor suppressive in clinical trials, but is limited by its inability to shrink or completely eliminate various types of tumors. Also, not all NF1 patients respond to the aforementioned therapeutic agents, and thus more therapeutic agents are needed.

Meanwhile, as a result of a survey on types of gene mutations in Korean neurofibromatosis patients, it was reported that about 30 % of patients had a stop-codon mutation. In addition, ataluren (PTC THERAPEUTICS, USA) with premature stop codon read-through activity has recently been studied as a therapeutic agent for muscle diseases in clinical studies, and has been mentioned as a potential therapeutic agent for neurofibromatosis patients having a stop-codon mutation, but there are no studies on the drug efficacy for ataluren.

### [Detailed Description of Invention]

### [Technical Problem]

One aspect is to provide a biomarker composition that can predict susceptibility of a neurofibromatosis patient to a compound having premature stop codon read-through activity by using Ras activity or p-ERK activity.

Another aspect is to provide a kit for predicting susceptibility of a neurofibromatosis patient to the compound, including the biomarker composition.

Another aspect is to provide a method of providing information for predicting susceptibility of a neurofibromatosis patient to the compound by using the biomarker composition.

Another aspect is to provide a pharmaceutical composition for preventing or treating neurofibromatosis, including a compound that can suppress Ras activity.

Another aspect is to provide a screening method by a therapeutic agent for neurofibromatosis, including: (a) contacting a biological sample having an NF1 gene mutation with a test substance; and (b) measuring, in the biological sample resulting from the contacting, an expression level of: (1) at least one protein selected from the group consisting of a GTP-bound and activated RAS (Ras-GTP) protein and an activated p-ERK protein; or (2) a gene encoding the at least one protein.

Another aspect is to provide use of a biomarker for predicting susceptibility to a therapeutic agent for neurofibromatosis, the biomarker including an agent that can measure an expression level of: (1) at least one protein selected from the group consisting of a GTP-bound and activated RAS (Ras-GTP) protein and an activated p-ERK protein; or (2) a gene encoding the at least one protein.

Another aspect is to provide a method of predicting susceptibility of a neurofibromatosis patient to a therapeutic agent for neurofibromatosis from a biological sample obtained from an individual, including measuring an expression level of: (1) at least one protein selected from the group consisting of a GTP-bound and activated RAS (Ras-GTP) protein and an activated p-ERK protein; or (2) a gene encoding the at least one protein.

Another aspect is to provide a method of preventing or treating neurofibromatosis, including administering a composition to an individual, the composition including, as an active ingredient, the compound or a pharmaceutically acceptable salt thereof:

Another aspect is to provide use of a composition for preparation of a therapeutic agent to be used in prevention or treatment of neurofibromatosis, the composition including, as an active ingredient, the compound or a pharmaceutically acceptable salt thereof.

### [Technical Solution]

To achieve the aforementioned objectives, one aspect provides a biomarker composition for predicting susceptibility to a therapeutic agent for neurofibromatosis, including an agent for measuring an expression level of: (1) at least one protein selected from the group consisting of a GTP-bound and activated RAS (Ras-GTP) protein and an activated p-ERK protein; or (2) a gene encoding the at least one protein.

The term "RAS protein" is a type of low-molecular-weight guanosine triphosphate (GTP)-binding protein, and may refer to a protein having isoforms such as a K-Ras protein, an H-Ras protein, an N-Ras protein, and the like. The Ras protein may be responsible for mediating signals of cell proliferation, differentiation, and adhesion upon various extracellular stimuli, from upstream tyrosine kinase receptors to downstream effectors.

The Ras protein is active when bound to GTP (Ras-GTP), and is inactive when bound to guanosine diphosphate (GDP) (Ras-GDP). By changing the states between active and inactive forms, the Ras protein may regulate cell proliferation. Meanwhile, the Ras protein is mutated at a high frequency in human cancer, and due to this mutation, the Ras protein is known to be always active.

The active Ras with transformation activity may amino acid mutations that are critical to interactions with the guanine ring or phosphate group of GDP or GTP, resulting in a decrease in the intrinsic GTP hydrolysis activity or an increase in the GDP dissociation rate. Depending on the expression of the active Ras, ratios of the GTP-bound active forms in cells may increase, causing abnormalities in the control of cell proliferation. Such GTP-bound and activated Ras may bind to Raf to directly control the serine (Ser)-threonine (Thr) protein kinase cascade from Raf to MAP protein kinases.

The ERK may be one of the Ser/Thr protein kinases regulated by the Ras protein. Inactivated ERK may be present in the cytoplasm, whereas activated ERK may translocate into the nucleus. In addition, activated ERK may regulate signal transduction systems by phosphorylating a target protein or activating other protein kinases.

The term "susceptibility" may indicate whether a particular drug exhibits a therapeutic effect in an individual neurofibromatosis patient. Even with types of therapeutic agents recognized to be effective in the treatment of neurofibromatosis, it is known that some therapeutic agents may or may not be effective for individual patients. As such, whether a therapeutic agent is effective for neurofibromatosis in individual patients may be referred to as susceptibility to a therapeutic agent. The term "susceptibility" may be used interchangeably with "treatment responsiveness," and wording "for predicting susceptibility" may be used interchangeably with "for predicting treatment responsiveness" or 'for companion diagnosis."

The term "companion diagnosis" may refer to a diagnostic technique for predicting treatment responsiveness of a patient.

The composition for companion diagnosis may be used to provide information on: the need for administration of a therapeutic agent for neurofibromatosis; the possible development of drug resistance to a therapeutic agent for neurofibromatosis; the sensitivity to a therapeutic agent for neurofibromatosis; or the prediction of prognosis of treatment with a therapeutic agent for neurofibromatosis.

The term "biomarker" may also be used as a companion diagnostic marker, and may refer to a substance in a biological sample that can predict in advance responsiveness of a patient to a specific drug treatment. The biomarker may include an organic biomolecule, such as a polypeptide or nucleic acid (e.g., mRNA, etc.), a lipid, a glycolipid, a glycoprotein, sugar (e.g., monosaccharide, disaccharide, oligosaccharide, etc.), and the like, which is increased or decreased in a biological sample obtained from a neurofibromatosis patient compared to a biological sample obtained from a normal individual.

In an embodiment, the biomarker for predicting susceptibility to a therapeutic agent for neurofibromatosis may be a GTP-bound and activated RAS (Ras-GTP) protein or a gene encoding the same. When the expression level of the Ras-GRP protein or the gene encoding the same is high, the susceptibility to a therapeutic agent for neurofibromatosis may be determined to be high, and conversely, when the expression level of the Ras-GRP protein or the gene encoding the same is low, the susceptibility to a therapeutic agent for neurofibromatosis may be determined to be low.

In an embodiment, the biomarker for predicting susceptibility to a therapeutic agent for neurofibromatosis may be an activated p-ERK protein or a gene encoding the same. When the expression level of the p-ERK protein or the gene encoding the same is high, the susceptibility to a therapeutic agent for neurofibromatosis may be determined to be high, and conversely, when the expression level of the p-ERK protein or the gene encoding the same is low, the susceptibility to a therapeutic agent for neurofibromatosis may be determined to be low.

The therapeutic agent for neurofibromatosis may be a biomarker composition for predicting susceptibility to the therapeutic agent for neurofibromatosis, which is a compound represented by Formula 1 or a pharmaceutically acceptable salt thereof:

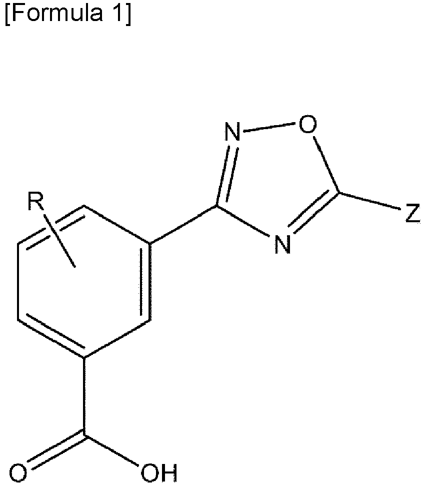

wherein, in the formula above,
Z may be halogen, hydroxyl, substituted or unsubstituted alkyl, substituted or unsubstituted alkoxy, substituted or unsubstituted aryl, substituted or unsubstituted heteroaryl, substituted or unsubstituted cycloalkyl, substituted or unsubstituted heterocycle, or substituted or unsubstituted heterocycloalkyl; and
R may be hydrogen or halogen.

The term "halogen" may refer to, unless otherwise specified, fluorine (F), chlorine (Cl), bromine (Br), or iodine (I).

The term "hydroxyl" may refer to, unless otherwise specified, a group of monovalent atoms consisting of one hydrogen atom and one oxygen atom, and may refer to a group with a formula -OH.

The term "alkyl" may refer to, unless otherwise specified, a linear or branched, saturated hydrocarbon residue. For example, "C₁₋₁₀ alkyl" may refer to alkyl with a skeleton consisting of 1 to 10 carbon atoms. For example, the C₁₋₁₀ alkyl may include methyl, ethyl, n-propyl, i-propyl, n-butyl, i-butyl, t-butyl, n-pentyl, i-pentyl, t-pentyl, sec-pentyl, neopentyl, hexyl, heptyl, octyl, nonyl, decyl, etc.

The term "alkoxy" may refer to, unless otherwise specified, a group with a formula -O-alkyl, where the alkyl group defined above is attached to the parent compound via an oxygen atom. An alkyl portion of an alkoxy group may have 1 to 20 carbon atoms (i.e., C₁-C₂₀ alkoxy), 1 to 12 carbon atoms (i.e., C₁-C₁₂ alkoxy), or 1 to 6 carbon atoms (i.e., C₁-C₆ alkoxy). Suitable examples of the alkoxy group may include methoxy (-O-CH₃ or - OMe), ethoxy (-OCH₂CH₃ or -OEt), t-butoxy (-O-C(CH₃)₃ or -O-tBu), etc.

The term "aryl" may refer to an aromatic hydrocarbon radical derived by removing one hydrogen atom from six carbon atoms of the parent aromatic ring system. For example, an aryl group may have 6 to 20 carbon atoms, 6 to 14 carbon atoms, or 6 to 12 carbon atoms.

The term "cycloalkyl" may refer to a saturated monocycle or polycycle, containing only carbon atoms in the ring. The cycloalkyl may have 3 to 7 carbon atoms in a monocycle, 7 to 12 carbon atoms in bicycloalkyl, and up to about 20 carbon atoms in a polycycle.

The term "heterocycle" may refer to an oriented or non-oriented ring including one or more heteroatoms, which may be either saturated or unsaturated and may be either monocyclic or polycyclic. For example, "4- to 10-membered heterocycle" may refer to a heterocycle with a skeleton consisting of 4 to 10 atoms, as well as with heteroatoms and carbon atoms. Specific examples of the 4- to 10-membered heterocycle may include azetidine, diazetidine, pyrrolidine, pyrrole, imidazolidine, imidazole, pyrazolidine, pyrazole, oxazolidine, oxazole, isoxazolidine, isoxazole, thiazolidine, thiazole, isothiazolidine, isothiazole, piperidine, pyridine, piperazine, diazine, morpholine, thiomorpholine, azepane, diazepane, etc.

The term "heteroaryl" may refer to aromatic heterocyclyl including one or more heteroatoms in the ring. Non-limiting examples of the heteroaryl may include pyridinyl, pyrrolyl, oxazolyl, indolyl, isoindolyl, purinyl, furanyl, thienyl, benzofuranyl, benzothiophenyl, carbazolyl, imidazolyl, thiazolyl, isoxazolyl, pyrazolyl, isothiazolyl, quinolyl, isoquinolyl, pyridazyl, pyrimidyl, pyrazyl, etc. (in which one or more substituents are included in the ring).

The term "heterocycloalkyl" may refer to non-aromatic heterocyclyl including one or more heteroatoms in the ring. The heterocycloalkyl may have one or more carbon-carbon double bonds or carbon-hetero atom double bonds in the ring to the extent that the ring is not aromatic due to the presence of such double bonds. Non-limiting examples of the heterocycloalkyl may include azetidinyl, aziridinyl, pyrrolidinyl, piperidinyl, piperazinyl, homopiperazinyl, morpholino, thiomorpholino, tetrahydrofuranyl, tetrahydrothiofuranyl, tetrahydropyranyl, pyranyl, etc. (in which one or more substituents may be included in the ring).

The term "heteroatom" may refer to an atom other than carbon (C), and a specific example thereof may include a nitrogen atom (N), an oxygen atom (O), or a sulfur atom (S).

The term "substitution" may refer to the replacement of a hydrogen atom in a molecular structure with a substituent such that the substitution results in a chemically stable compound without exceeding the valence of the designated atom. For example, the wording "a group A is substituted with a substituent B" may refer that a hydrogen atom bonded to an atom such as carbon constituting the skeleton of the group A is replaced by the substituent B, resulting in the formation of a covalent bond between the group A and the substituent B.

Z may be: p-tolyl; (4-chloromethyl-phenyl); (2-chloro-pyridin-3-yl); (2-fluoro-phenyl); (3,4-difluoro-phenyl); (4-methoxy-phenyl); benzo[1,3]dioxol-yl; (4-ethyl-phenyl); o-tolyl; (2-chloro-phenyl); (3-methyl-thiophen-2-yl); benzo[b]thiophen-2-yl; (3-fluoro-phenyl); (4-tert-butyl-phenyl); (2-methoxy-phenyl); (2,difluoro-phenyl); thiophen-2-yl; (2,4-difluoro-phenyl); (3-chloro-phenyl); m-tolyl; (4-trifluoromethyl-phenyl); (4-fluoro-phenyl); (3-methoxy-phenyl); phenyl; (2,6-difluoro-phenyl); (2,dimethyl-furan-3-yl); (4-pyrrol-1-yl-phenyl); (3-dimethylamino-phenyl); biphenyl-4-yl; (4-dimethylamino-phenyl); benzo[1,2,5]oxadiazol-yl; m-tolyl; (2-trifluoromethyl-phenyl); (6-chloro-pyridin-3-yl); (3,bis-trifluoromethyl-phenyl); furan-2-yl; (4-nitro-phenyl); (3,4-dimethoxy-phenyl); (3-trifluoromethoxy-phenyl); naphthalen-1-yl; cyclohexyl; pyridin-3-yl; pyridin-4-yl; cyclopentyl; cyclopropyl; (4-pentyloxy-phenyl); (3,4,trimethoxy-phenyl); (4-isobutylphenyl); cyclobutyl; (1-acetyl-piperidin-4-yl); isoxazol-yl; [2-chloro-6-fluoro-phenyl)-methyl-isoxazol-4-yl], or [2-chloro-phenyl)-methyl-isoxazol-4-yl]. Specifically, Z may be: (2-fluoro-phenyl); (3,4-difluoro-phenyl); (2-chloro-phenyl); (3-fluoro-phenyl); (2,5-difluoro-phenyl); (2,4-difluoro-phenyl); (3-chloro-phenyl); (4-fluoro-phenyl); or (2,6-difluoro-phenyl).

The compound represented by Formula 1 may have the premature stop codon read-through activity.

The compound represented by Formula 1 may be 3-[5-(2-fluoro-phenyl)-[1,2,4]oxadiazol-3-yl]-benzoic acid. The compound may be represented by Formula 2:

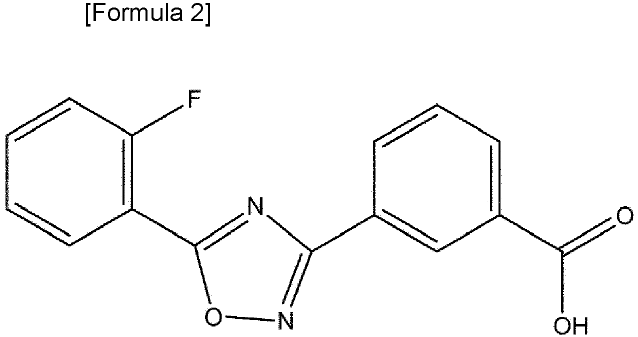

The compound represented by Formula 2 is also called ataluren, PCT124, or Translarna, which has the premature stop codon read-through activity.

The therapeutic agent for neurofibromatosis may include a pharmaceutically acceptable salt of the compound. The pharmaceutically acceptable salt should have low toxicity to humans, and may not have any negative effect on the biological activity and physicochemical properties of the parent compound.

For example, the pharmaceutically acceptable salt may be an alkali metal salt (sodium salt, etc.) or an alkaline earth metal salt (potassium salt, etc.). The alkali metal salt or alkaline earth metal salt may be, for example, obtained by dissolving the compound in a solution containing an excess of alkali metal hydroxide or alkaline earth metal hydroxide, filtering salts of the compound that has not been dissolved, and then drying the filtrate by evaporation.

In addition, the compound may have a chiral carbon center, and thus may exist in the form of an R or S isomer, a racemic compound, an individual enantiomer or mixture, an individual diastereomer, or a mixture, wherein all such stereoisomers and mixtures thereof may fall within the scope of the present disclosure.

In addition, the compound may include hydrates and solvates of the compound. The hydrates and solvates may be prepared by using known methods, and may preferably be non-toxic and water-soluble. In particular, preferably, the hydrates and solvates may each be a combination of 1 to 5 molecules of water and an alcoholic solvent (particularly, ethanol, etc.).

Measuring the expression level of the Ras-GTP protein and/or the activated p-ERK protein is a process of confirming the presence and expression level of the protein in a biological sample of an individual to predict susceptibility to the therapeutic agent for neurofibromatosis. Here, the amount of the protein may be confirmed by using a molecule that binds specifically to the protein.

An agent that can measure the expression level of the protein may include one or more selected from the group consisting of a monoclonal antibody, a polyclonal antibody, a chimeric antibody, a ligand, peptide nucleic acid (PNA), an aptamer, and a nanoparticle, but is not limited thereto.

Analysis methods for this measurement may include western blotting, enzyme linked immunosorbent assay (ELISA), radioimmunoassay (RIA), radioimmunodiffusion, Ouchterlony immunodiffusion, rocket immunoelectrophoresis, immunoprecipitation assay, immunohistochemical analysis, complement fixation assay, fluorescence activated cell sorter (FACS), protein chip, two-dimensional electrophoresis analysis, protein mass spectrometry, liquid chromatography-mass spectrometry (LC-MS), liquid chromatography-mass spectrometry/mass spectrometry (LC-MS/MS), matrix desorption/ionization time of flight mass spectrometry (MALDI-TOF), surface enhanced laser desorption/ionization time of flight mass spectrometry (SELDI-TOF), etc., but are not limited thereto.

According to an embodiment, the agent that can measuring the expression level of the Ras-GTP protein may be one that binds specifically to the Ras-GTP protein but does not bind to the Ras-GDP protein.

According to an embodiment, the agent that can measuring the expression level of the activated p-ERK protein may be one that binds specifically to the p-ERK protein.

Measuring the expression level of the gene encoding the aforementioned protein is a process of confirming the presence and expression level of the gene encoding the aforementioned protein in a biological sample of an individual to predict susceptibility to the therapeutic agent for neurofibromatosis. Here, the amount of the gene encoding the aforementioned protein may be confirmed by using a molecule that binds specifically to the gene encoding the aforementioned protein.

An agent that can measure the expression level of the gene encoding the aforementioned protein may be one or more selected from the group consisting of a primer pair, a probe, and an antisense nucleotide that binds specifically to the gene, but is not limited thereto.

Analysis methods for this measurement may include reverse transcriptase-polymerase chain reaction (RT-PCR), real time-polymerase chain reaction, RNase protection assay (RPA), northern blotting, DNA chips, etc., but are not limited thereto.

The neurofibromatosis may be characterized by a stop codon mutation in the NF1 gene.

The term "stop codon mutation" may refer that a part of the base sequence becomes one of stop codons such as UAG, UAA, and UAG due to a mutation, resulting in interruption of the synthesis of a polypeptide.

The stop codon mutation may be caused by a nonsense mutation, which is one of point mutations in which a DNA base is replaced with another base, or by a frameshift mutation.

The term "nonsense mutation" may refer to a mutation in which the synthesis of proteins no longer occurs when DNA base sequences are converted into proteins and a part of the base sequences is converted to a stop codon (one of UAG, UAA, and UGA).

The term "frameshift mutation" may refer a mutation by insertion or deletion of one or two nucleotides or nucleotides that is not a multiple of three in the DNA base sequences, resulting in a significant change in the arrangement of amino acids in a protein produced through transcription and translation of a gene.

In an embodiment, the stop codon mutation may be i) conversion of arginine (Arg, R), the 1748th amino acid constituting the NF1 protein, to a stop codon by substitution of cytidine (C) for thymine (T) at base 5242 in the DNA sequence of the NF1 gene, ii) conversion of glutamine (Gln, Q), the 854th amino acid constituting the NF1 protein, to a stop codon by substitution of cytidine (C) for thymine (T) at base 2560 in the DNA sequence of the NF1 gene, iii) conversion of arginine (Arg, R), the 1513rd amino acid constituting the NF1 protein, to a stop codon by substitution of cytidine (C) for thymine (T) at base 4537 in the DNA sequence of the NF1 gene, iv) conversion of leucine (Leu, L), the 993rd amino acid constituting the NF1 protein, to a stop codon by substitution of thymine (T) for guanine (G) at base 2978 in the DNA sequence of the NF1 gene, or v) conversion of arginine (Arg, R), the 2496th amino acid constituting the NF1 protein, to a stop codon by substitution of cytidine (C) for thymine (T) at base 7486 in the DNA sequence of the NF1 gene.

In another embodiment, neurofibromatosis patients who carry the stop codon mutation in the NF1 gene are screened, and the Ras-GTP activity in fibroblasts is measured from the screened patients. Then, as a result of the treatment with ataluren, the expression of the Ras-GTP protein and the p-ERK protein is significantly decreased in a group of patients where the Ras-GTP expression is significantly increased compared to normal individuals, confirming that ataluren is effective in the treatment of neurofibromatosis. In this regard, among neurofibromatosis patients with the stop codon mutation in the NF1 gene, the susceptibility to ataluren may be predicted based on whether the expression of the active Ras (Ras-GTP) protein and/or the activated p-ERK protein is increased or decreased.

Another aspect provides a kit for predicting susceptibility to a therapeutic agent for neurofibromatosis, including the biomarker composition.

The kit may enable to predict susceptibility to the therapeutic agent for neurofibromatosis by using a biological sample separated from an individual. In detail, the kit may enable to predict susceptibility to the therapeutic agent for neurofibromatosis by using fibroblasts collected from an individual.

The kit may include not only preparations for measuring the expression level of the protein or the gene, but also tools, reagents, etc. commonly used in immunological assays.

Examples of the tools or reagents may include a suitable carrier, a labeling substance capable of generating a detectable signal, a chromophore, a solubilizer, a detergent, a buffer, a stabilizer, etc., but are not limited thereto. When the labeling substance is an enzyme, a substrate and a reaction stopper that can measure the enzyme activity may be also included. The carrier may be a water-soluble carrier or a water-insoluble carrier. An example of the water-soluble carrier may include a physiologically acceptable buffer known in the art, such as PBS, and examples of the water-insoluble carrier may include polystyrene, polyethylene, polypropylene, polyester, polyacrylonitrile, a fluororesin, cross-linked dextran, polysaccharide, a polymer such as magnetic fine particle plated with metal on latex, other paper, glass, metal, agarose, and a combination thereof.

The biomarker composition is the same as described herein.

Another aspect provides a method of providing information for predicting susceptibility to the therapeutic agent for neurofibromatosis from a biological sample obtained from an individual, including measuring an expression level of: (1) at least one protein selected from the group consisting of a GTP-bound and activated RAS (Ras-GTP) protein and an activated p-ERK protein; or (2) a gene encoding the at least one protein.

The method may further include determining the susceptibility to the therapeutic agent for neurofibromatosis to be high when the expression level of the activated Ras-GTP protein or the gene encoding the same is increased compared to the expression level measured in a biological sample obtained from normal individuals, and conversely, determining the susceptibility to the therapeutic agent for neurofibromatosis to be low when the expression level is low.

The method may further include determining the susceptibility to the therapeutic agent for neurofibromatosis to be high when the expression level of the activated p-ERK protein or the gene encoding the same is increased compared to the expression level measured in a biological sample obtained from normal individuals, and conversely, determining the susceptibility to the therapeutic agent for neurofibromatosis to be low when the expression level is low.

The term "biological sample" may refer to all samples obtained from an individual, where the expression level of: the Ras-GTP protein or the gene encoding the same according to an aspect; and/or the p-ERK protein or the gene encoding the same according to an aspect can be measured.

The biological sample may be blood, plasma, serum, urine, saliva, sputum, cerebrospinal fluid, cells, cell culture, tissue extract, or tumor tissue, but is not limited thereto. In detail, the biological sample may be fibroblasts collected from an individual. The biological sample may be prepared by treatments according to methods commonly used in the art.

Another aspect provides a pharmaceutical composition for preventing or treating neurofibromatosis, including, as an active ingredient, a compound represented by Formula 1 or a pharmaceutically acceptable salt thereof:

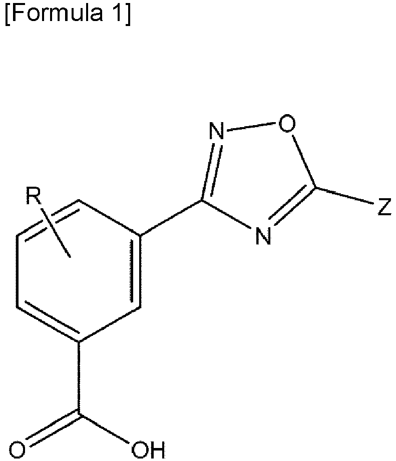

wherein, in the formula above,
Z may be halogen, hydroxyl, substituted or unsubstituted alkyl, substituted or unsubstituted alkoxy, substituted or unsubstituted aryl, substituted or unsubstituted heteroaryl, substituted or unsubstituted cycloalkyl, substituted or unsubstituted heterocycle, or substituted or unsubstituted heterocycloalkyl; and
R may be hydrogen or halogen.

The compound represented by Formula 1 and the pharmaceutically acceptable salt thereof are the same as described herein.

The compound represented by Formula 1 may be 3-[5-(2-fluoro-phenyl)-[1 ,2,4]oxadiazol-3-yl]-benzoic acid.

The neurofibromatosis may be characterized by a stop codon mutation in the NF1 gene.

The neurofibromatosis may be also characterized by increased activity of the Ras protein.

The neurofibromatosis is the same as described herein.

In an embodiment, neurofibromatosis patients who carry the stop codon mutation in the NF1 gene are screened, and the Ras-GTP activity in fibroblasts is measured from the screened patients. Then, as a result of the treatment with ataluren, the activity of the Ras-GTP protein and the p-ERK protein is significantly decreased in a group of patients where the Ras-GTP expression is significantly increased compared to normal individuals, confirming that ataluren is effective in the treatment of neurofibromatosis. In this regard, ataluren may be used to treat neurofibromatosis patients who have the stop codon mutation in the NF1 as well as the increased expression of the active Ras (Ras-GTP).

The pharmaceutical composition may include the compound as an active ingredient in an amount of about 0.1 wt% to about 90 wt%, for example, about 0.5 wt% to about 75 wt%, and for example, about 1 wt% to about 50 wt%, based on the total weight of the composition.

The pharmaceutical composition may include conventional, non-toxic, pharmaceutically acceptable additives that are blended in the form of preparations according to conventional methods. For example, the pharmaceutical composition may further include a pharmaceutically acceptable carrier, diluent, or excipient.

Examples of additives used in the pharmaceutical composition may include a sweetener, a binder, a solvent, a dissolution aid, a wetting agent, an emulsifier, an isotonic agent, an absorbent, a disintegrant, an antioxidant, a preservative, a lubricant, a glidant, a filler, a flavoring agent, etc. For example, the additives may include lactose, dextrose, sucrose, mannitol, sorbitol, cellulose, glycine, silica, talc, stearic acid, stearin, magnesium stearate, magnesium aluminosilicate, starch, gelatin, tragacanth gum, alginic acid, sodium alginate, methylcellulose, sodium carboxymethylcellulose, agar, water, ethanol, polyethylene glycol, polyvinylpyrrolidone, sodium chloride, calcium chloride, orange essence, strawberry essence, vanilla flavor, etc.

The pharmaceutical composition may be blended in the form of various preparations for oral administration (e.g., a tablet, a pill, powder, a capsule, a syrup, or an emulsion) or for parenteral administration (e.g., an intramuscular, intravenous, or subcutaneous injection).

In detail, the pharmaceutical composition may be blended in the form of preparations for oral administration, and additives used herein may include cellulose, calcium silicate, corn starch, lactose, sucrose, dextrose, calcium phosphate, stearic acid, magnesium stearate, calcium stearate, gelatin, talc, surfactants, suspending agents, emulsifiers, diluents, etc. In addition, examples of the glidant may include colloidal silicon dioxide, magnesium silicate, etc.; examples of diluents may include microcrystalline cellulose, lactose anhydrous, lactose monohydrate, silicified MCC HD 90, etc.; examples of the disintegrant may include croscarmellose sodium, crospovidone, etc.; and examples of the lubricant may include magnesium stearate, sodium lauryl sulfate, stearic acid, and etc.

In addition, liquid preparations for oral administration may include a suspension, an emulsion, a syrup, etc., and may include various excipients such as a wetting agent, a sweetening agent, a flavor, a preservative, and the like, in addition to commonly used simple diluents such as water and liquid paraffin.

In addition, preparations for parenteral administration may include a sterile aqueous solution, a non-aqueous solvent, a suspension, an emulsion, a lyophilized preparation, and a suppository. Examples of the non-aqueous solvent and suspension may include propyleneglycol, polyethylene glycol, plant oil such as olive oil, injectable ester such as ethyloleate, etc. For use as a base of the suppository, witepsol, macrogol, and twin 61, cacao butter, Laurin, glycerogeratin, etc. may be used. Meanwhile, the injection may include conventional additives such as a solubilizer, an isotonic agent, a suspending agent, an emulsifier, a stabilizer, a preservative, and the like.

The pharmaceutical composition may be administered to a patient in a therapeutically effective amount or in a pharmaceutically effective amount.

The term "therapeutically effective amount" or "pharmaceutically effective amount" may refer to an amount of a compound or composition that is effective in preventing or treating a target disease, the amount being sufficiently enough to treat the disease at a reasonable benefit/risk ratio applicable to the medical treatment and being unlikely to cause a side effect. Levels of the effective amount may be determined by factors including health conditions of a patient, types and severity of a disease, activity of a drug, sensitivity to a drug, administration methods, administration periods, administration routes and release rates, duration of treatment, drugs used in combination or concurrently, and other factors well known in the medical field.

The pharmaceutical composition may be administered as an individual therapeutic agent, administered in combination with other therapeutic agents, administered sequentially or simultaneously with a conventional therapeutic agent, and administered once or multiple times. Considering all of the aforementioned factors, it is important to administer an amount for achieving a maximum effect in a minimum amount without side effects, and such an amount may be easily determined by those skilled in the art.

In detail, the effective amount of the compound in the pharmaceutical composition may vary depending on the age, gender, and weight of a patient, and in general, about 0.1 mg to about 1,000 mg per kg of the body weight or about 5 mg to about 200 mg per kg of the body weight may be administered daily or every other day, or divided into one to three daily doses. However, depending on the administration routes, severity of a disease, and gender, weight, and age of a patient, etc., the effective amount may increase or decrease, and thus the range is not limited thereto.

In addition, the pharmaceutical composition may be administered for tumor therapy in combination with chemotherapy, radiation therapy, immunotherapy, hormone therapy, bone marrow transplantation, stem cell replacement therapy, other biological treatments, surgical interventions, or combinations thereof. For example, the pharmaceutical composition may be used as adjuvant therapy together with other long-term treatment strategies, or may be used to maintain conditions of a patient after tumor regression or chemopreventive therapy in critically ill patients.

Another aspect provides a screening method by the therapeutic agent for neurofibromatosis, including: (a) contacting a biological sample having an NF1 gene mutation with a test substance; and (b) measuring, in the biological sample resulting from the contacting, an expression level of: (1) at least one protein selected from the group consisting of a GTP-bound and activated RAS (Ras-GTP) protein and an activated p-ERK protein; or (2) a gene encoding the at least one protein.

The biological sample may be a cell or an animal model. The sample may by a sample separated from a neurofibromatosis patient.

According to an embodiment, the screening method may further include, when the expression level of the Ras-GTP protein or the gene encoding the same in the biological sample after the contact with a test substance is decreased compared to the expression level before the contact with a test substance, determining whether the test substance is a therapeutic agent for neurofibromatosis.

According to an embodiment, the screening method may further include, when the expression level of the p-ERK protein or the gene encoding the same in the biological sample after the contact with a test substance is decreased compared to the expression level before the contact with a test substance, determining whether the test substance is a therapeutic agent for neurofibromatosis.

The screening method may further include comparing the results of measuring the expression level of the Ras-GTP protein or the gene encoding the same in the biological sample that is in contact with the test substance with the results measured in a biological sample that is not in contact with the test substance.

The screening method may further include comparing the results of measuring the expression level of the p-ERK protein or the gene encoding the same in the biological sample that is in contact with the test substance with the results measured in a biological sample that is not in contact with the test substance.

Another aspect provides use of a biomarker for predicting susceptibility to a therapeutic agent for neurofibromatosis, the biomarker including an agent that can measure an expression level of: (1) at least one protein selected from the group consisting of a GTP-bound and activated RAS (Ras-GTP) protein and an activated p-ERK protein; or (2) a gene encoding the at least one protein.

Another aspect provides a method of predicting susceptibility to a therapeutic agent for neurofibromatosis in a neurofibromatosis patient from a biological sample obtained from an individual, including measuring an expression level of: (1) at least one protein selected from the group consisting of a GTP-bound and activated RAS (Ras-GTP) protein and an activated p-ERK protein; or (2) a gene encoding the at least one protein.

Another aspect provides a method of preventing or treating neurofibromatosis, including administering a composition to an individual, the composition including, as an active ingredient, a compound represented by Formula 1 or a pharmaceutically acceptable salt thereof:

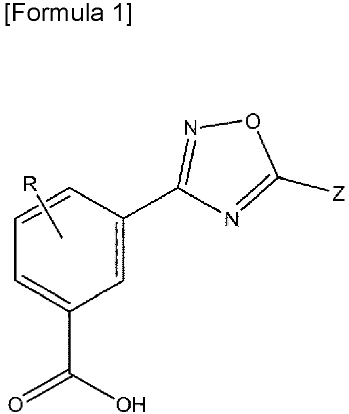

wherein, in the formula above,
Z may be halogen, hydroxyl, substituted or unsubstituted alkyl, substituted or unsubstituted alkoxy, substituted or unsubstituted aryl, substituted or unsubstituted heteroaryl, substituted or unsubstituted cycloalkyl, substituted or unsubstituted heterocycle, or substituted or unsubstituted heterocycloalkyl; and
R may be hydrogen or halogen.

Another aspect provides use of a composition for preparation of a therapeutic agent to be used in prevention or treatment of neurofibromatosis, the composition including, as an active ingredient, a compound represented by Formula 1 or a pharmaceutically acceptable salt thereof: 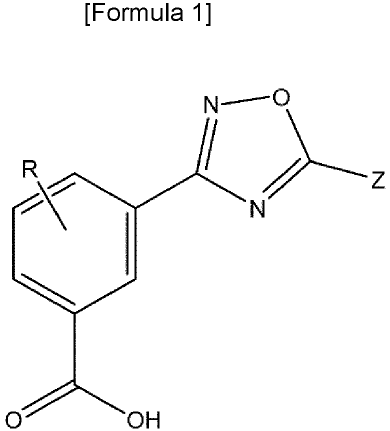
wherein, in the formula above,
Z may be halogen, hydroxyl, substituted or unsubstituted alkyl, substituted or unsubstituted alkoxy, substituted or unsubstituted aryl, substituted or unsubstituted heteroaryl, substituted or unsubstituted cycloalkyl, substituted or unsubstituted heterocycle, or substituted or unsubstituted heterocycloalkyl; and
[R may be hydrogen or halogen.

Redundancies are omitted in consideration of the complexity of the present specification, and terms not otherwise defined herein may have the meanings commonly used in the technical field to which the present disclosure pertains.

### [Advantageous Effects]

The biomarker composition for predicting susceptibility to a therapeutic agent for neurofibromatosis according to an aspect may be used to accurately diagnose susceptibility to a therapeutic agent for neurofibromatosis by a simple method of measuring an expression level of: a GTP-bound and activated RAS (Ras-GTP) protein or a gene encoding the same in a biological sample of an individual; and/or an activated p-ERK protein or a gene encoding the same.

The pharmaceutical composition for preventing or treating neurofibromatosis according to an aspect inhibits the activity of the Ras protein, and thus may be usefully in the prevention or treatment of neurofibromatosis in which the activity of the Ras protein is increased.

### [Description of Drawings]

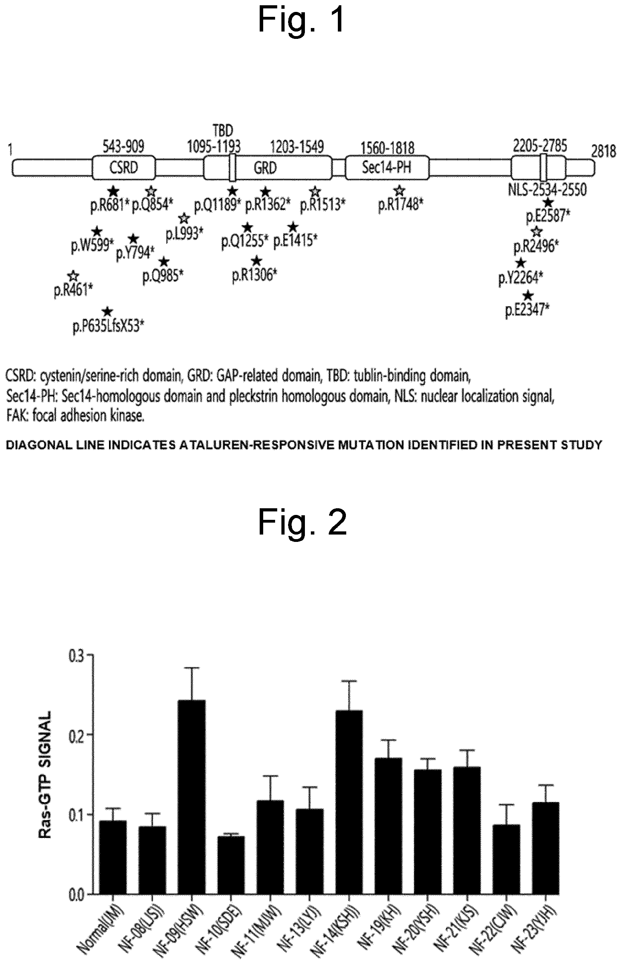

FIG. 1 is a diagram showing the information of stop codon mutations in a neurofibromatosis patient screened for having a stop codon mutation in the NF1 gene according to an aspect.
FIG. 2 shows the results of measuring the expression level of a GTP-bound and activated RAS (Ras-GTP) protein in a neurofibromatosis patient screened for having a stop codon mutation in the NF1 gene according to an aspect.

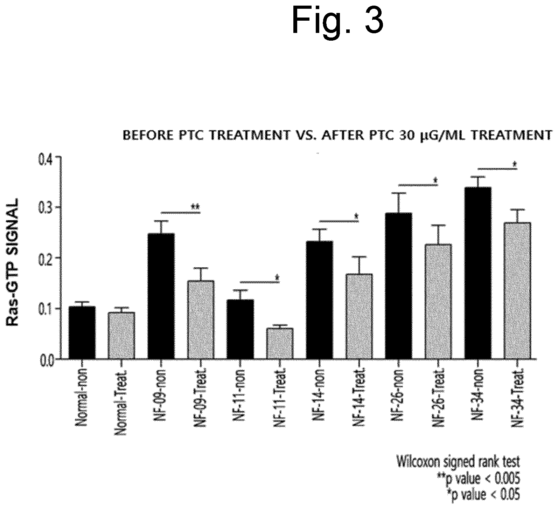

FIG. 3 shows the results of measuring changes in the expression of Ras-GTP before and after administration of 30 µg/ml of ataluren (PTC) to fibroblasts of a patient with a high expression level of Ras-GTP among neurofibromatosis patients screened as having a stop codon mutation in the NF1 gene according to an aspect.

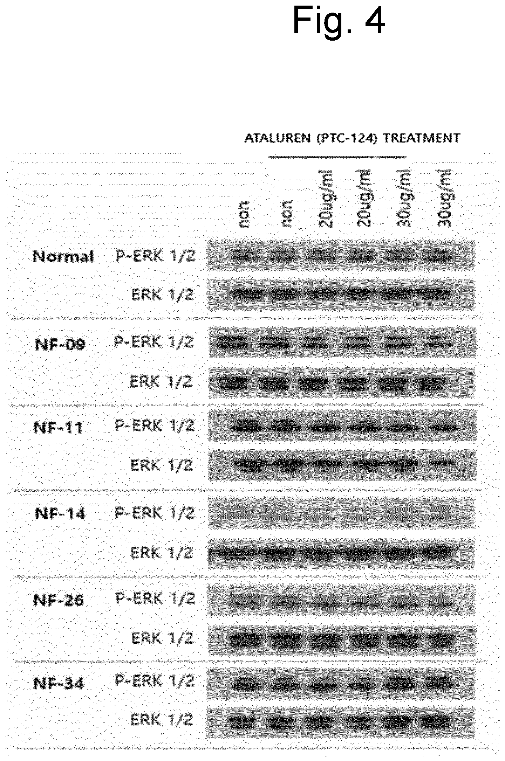

FIG. 4 shows the results of electrophoresis performed to compare expression levels of p-ERK before and after administration of 20 µg/ml or 30 µg/ml of ataluren (PTC) to fibroblasts of a patient with a high expression level of Ras-GTP among neurofibromatosis patients screened for having a stop codon mutation in the NF1 gene according to an aspect.

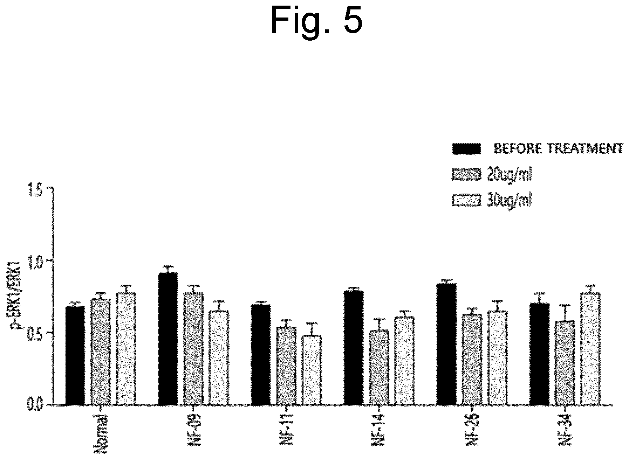

FIG. 5 shows the results of measuring expression ratios of ERK and p-ERK before and after administration of 20 µg/ml or 30 µg/ml of ataluren (PTC) to fibroblasts of a patient with a high expression level of Ras-GTP among neurofibromatosis patients screened for having a stop codon mutation in the NF1 gene according to an aspect.

### [Best Mode]

Hereinafter, the present disclosure will be explained in more detail by Examples. However, these Examples are for illustrative purposes only, and the scope of the present disclosure is not limited thereto.

### Example 1. Screening of patients having stop codon mutation in NF1 gene from neurofibromatosis patients

By using the DNA extracted from neurofibromatosis patients, the NF1 gene which is the causative gene of neurofibromatosis was amplified by polymerase chain reaction (PCR) and then subjected to Sanger sequencing. Based on the analysis results, 22 patients having a stop codon mutation in the NF1 gene were screened. The information on the stop codon mutation present in the NF1 gene of the screened patients is shown in Table 1 (FIG. 1).

**[Table 1]**

| ID | Initial | Mutation of *NF1* | |
|---|---|---|---|
| NF-08 | LJS | c.7759G>T | p.Glu2587Ter (p.Q2587*) |
| NF-09* | HSW | c.5242C>T | p.Arg1748Ter (p.R1748*) |
| NF-10 | SDE | c.1381C>T | p.Arg461Ter (p.R461*) |
| NF-11* | MJW | c.2560C>T | p.Gln854Ter (p.Q854*) |
| NF-13 | LYJ | c.7039G>T | p.Glu234Ter (p.E2347*) |
| NF-14* | KSH | c.4537C>T | p.Arg1513Ter (p.R1513*) |
| NF-19 | KH | c.4084C>T | p.Arg1362Ter (p.R1362*) |
| NF-20 | YSH | c.4243G>T | p.Glu1415Ter (p.E1415*) |
| NF-21 | KJS | c.3916C>T | p.Arg1306Ter (p.R1306*) |
| NF-22 | CJW | c.1381C>T | p.Arg461Ter (p.R461*) |
| NF-23 | YJH | c.4537C>T | p.Arg1513Ter (p.R1513*) |
| NF-24 | PJS | | p.Gln1255Ter (p.Q1255*) |
| NF-25 | PCJ | c.6792C>A | p.Tyr2264Ter (p.Y2264*) |
| NF-26* | KJW | c.2978T>G | p.Leu993Ter (p.L993*) |
| NF-27 | KDH | | p.Gln985Ter (p.Q985*) |
| NF-28 | KN | c.1797G>A | p.Trp599Ter (p.W599*) |
| NF-29 | HKW | c.6792C>A | p.Tyr2264Ter (p.Y2264*) |
| NF-30 | NYS | c.3916C>T | p.Arg1306Ter (p.R1306*) |
| NF-31 | HKH | c.3565C>T | p.Gln1189Ter (p.Q1189*) |
| NF-32 | KDH | c.1904del | p.Pro635Leufs*53 (p. P635LfsX53) |
| NF-33 | LYJ | c.2382T>A | p.Tyr794Ter (p.Y794*) |
| NF-34* | PCK | c.7486C>T | p.Arg2496Ter (p.R2496*) |

### Example 2. Measurement of activity of RAS protein in screened neurofibromatosis patients

The activity of the RAS protein was compared between the skin fibroblasts from normal individuals and the neurofibromatosis patients screened in Example 1 as follows.

The skin fibroblasts from donated normal individuals and neurofibromatosis patients were cultured in a DMEM supplemented with 10 % FBS and 1 % penicillin-streptomycin. When the cells were cultured until the density reached 70 % to 80 % of a container, the medium was replaced with an FBS-free medium, and the cells were cultured and subjected to a starvation process for 24 hours. Next, the cells were transferred to an FBS-containing medium, treated with epidermal growth factor (EGF), which is a Ras activator, by dilution to a concentration of 100 ng/ml, and then allowed for a reaction at 37 °C for 5 minutes to prepare each sample. To analyze the activity of the RAS proteins, a G-LISA Ras activation assay biochem Kit of Cytoskeleton Company (Absorbance Based) (Cytoskeleton, Cat No. BK131), which enables the measurement of a small number of cells and a large number of cells at the same time by compensating for the disadvantages of the pull-down assay, was used.

Briefly, in a method of analyzing the activity of the RAS proteins, cells were lysed by using a cell lysis solution included in the kit after treatment with EGF, only the supernatant was obtained, and the proteins contained in the supernatant were quantified. The proteins in the same amount from all samples were added to wells of a plate to which Ras-GTP binding protein was bound, and then a reaction was allowed therein at 4 °C for 30 minutes while shaking the plate at a rate of 300 rpm on a stirrer. Afterwards, unbound proteins were removed from the mixture after washing was performed thereon three times by using a washing solution, and anti-Ras antibodies were added thereto and allowed for a reaction in a stirrer at a rate of 300 rpm for 45 minutes at room temperature. Then, washing was performed thereon three times. After completely removing the washing solution, secondary antibodies were added and allowed for a reaction in a stirrer at a rate of 400 rpm for 45 minutes at room temperature for the same procedure as the reaction with the primary antibodies. Then, washing was performed thereon three times. Through these procedures, GTP-bound Ras proteins in the active state remained, whereas GDP-bound Ras proteins in the inactive state were removed by the washing. Next, an HRP detection reagent was added and a reaction was allowed for 15 minutes at room temperature to measure the absorbance at 490 nm. Accordingly, based on the amount of existing Ras-GTP, the activity of Ras proteins may be measured.

As a result, based on the Ras-GTP signals measured higher in most fibroblasts derived from neurofibromatosis patients than in normal individuals, it was confirmed that the activity of Ras proteins was increased (FIG. 2).

### Example 3. Measurement of changes in activity of RAS protein upon Ataluren treatment

The skin fibroblasts from donated normal individuals and neurofibromatosis patients were treated with Ataluren at concentrations of 20 ug/ml and 30 ug/ml and cultured, in a DMEM supplemented with 10 % FBS and 1 % penicillin-streptomycin. When the cells were cultured until the density reached 70 % to 80 % of a container, the medium was replaced with an FBS-free medium, and the cells were cultured and underwent a starvation process for 24 hours. Next, the cells were transferred to an FBS-containing medium, treated with EGF, which is a Ras activator, by dilution to a concentration of 100 ng/ml, and then allowed for a reaction at 37 °C for 5 minutes to prepare each sample. Measurement of the activity of Ras proteins in each sample was performed in the same manner as described in Example 2.

As a result, when Ataluren was treated on the skin fibroblasts of neurofibromatosis patients who have increased activity of Ras proteins compared to normal individuals, it was confirmed that the signal of Ras-GTP was significantly decreased (FIG. 3). Accordingly, it was found that Ataluren had a therapeutic effect on neurofibromatosis by reducing the activity of Ras proteins.

### Example 4. Measurement of change in expression level of p-ERK/ERK protein upon treatment with Ataluren

The same protein of which the Ras activity was measured in Example 3 was subjected to electrophoresis by using NuPAGETM 4-12% Bris-Tris Gel (Invitrogen).

As a result, it was confirmed that there was no change in the expression of phosphorylated ERK (p-ERK) in the active state in the fibroblasts of normal individuals upon the treatment with Ataluren, whereas the expression of p-ERK was decreased in the fibroblasts of neurofibromatosis patients in which the activity of Ras proteins has been decreased (FIG. 4).

To more accurately compare the ERK activity, the electrophoresed proteins were transferred to a hybond-ECL membrane, and the membrane was blocked with 3 % skim milk. Next, ERK and p-ERK antibodies were reacted on the membrane at 4 °C for 24 hours, and then washed by using TBST three times. Secondary antibodies were added thereto and allowed for a reaction at room temperature for 1 hour, and then washing was performed thereon by using TBST. The expression ratios of ERK and p-ERK in the washed membrane were measured.

As a result, it was confirmed that the expression level of p-the ERK protein was slightly increased in the fibroblasts of normal individuals upon the treatment with Ataluren, whereas the expression of p-ERK was decreased in the fibroblasts of neurofibromatosis patients in which the activity of Ras proteins has been decreased (FIG. 5).

## Claims

1. A biomarker composition for predicting susceptibility to a therapeutic agent for neurofibromatosis, comprising an agent for measuring an expression level of:
(1) at least one protein selected from the group consisting of a GTP-bound and activated RAS (Ras-GTP) protein and an activated p-ERK protein; or
(2) a gene encoding the at least one protein

2. The biomarker composition of claim 1, wherein the therapeutic agent for neurofibromatosis includes a compound represented by Formula 1 or a pharmaceutically acceptable salt thereof: 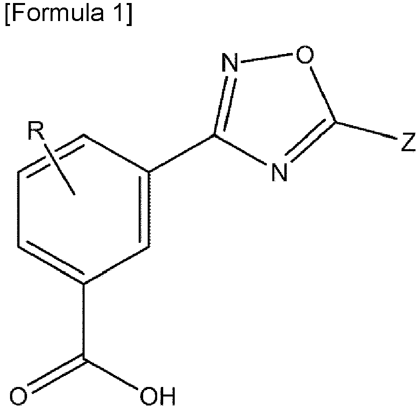
wherein, in the formula above,
Z is halogen, hydroxyl, substituted or unsubstituted alkyl, substituted or unsubstituted alkoxy, substituted or unsubstituted aryl, substituted or unsubstituted heteroaryl, substituted or unsubstituted cycloalkyl, substituted or unsubstituted heterocycle, or substituted or unsubstituted heterocycloalkyl; and
R is hydrogen or halogen.

3. The biomarker composition of claim 2, wherein the compound represented by Formula 1 is 3-[5-(2-fluoro-phenyl)-[1,2,4]oxadiazol-3-yl]-benzoic acid.

4. The biomarker composition of claim 1, wherein the agent for measuring the expression level of the at least one protein is selected from the group consisting of a monoclonal antibody, a polyclonal antibody, a chimeric antibody, a ligand, peptide nucleic acid (PNA), an aptamer, and a nanoparticle that bind specifically to the at least one protein.

5. The biomarker composition of claim 1, wherein the agent for measuring the expression level of the at least one protein is selected from the group consisting of a primer pair, a probe, and an antisense nucleotide that bind specifically to the at least one protein.

6. The biomarker composition of claim 1, wherein the neurofibromatosis is **characterized by** a stop codon mutation in an NF1 gene.

7. A kit for predicting susceptibility to a therapeutic agent for neurofibromatosis, comprising the biomarker composition of any one of claims 1 to 6.

8. A method of providing information for predicting susceptibility to a therapeutic agent for neurofibromatosis from a biological sample obtained from an individual, comprising measuring an expression level of:
(1) at least one protein selected from the group consisting of a GTP-bound and activated RAS (Ras-GTP) protein and an activated p-ERK protein; or
(2) a gene encoding the at least one protein.

9. A pharmaceutical composition for preventing or treating neurofibromatosis, comprising, as an active ingredient, a compound represented by Formula 1 or a pharmaceutically acceptable salt thereof: 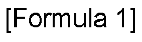 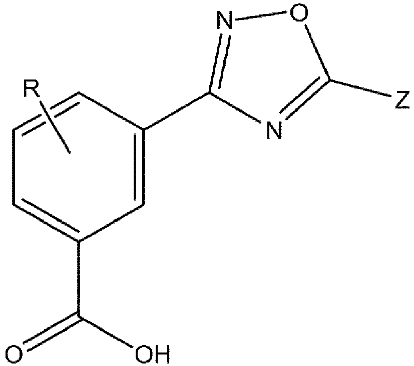
wherein, in the formula above,
Z is halogen, hydroxyl, substituted or unsubstituted alkyl, substituted or unsubstituted alkoxy, substituted or unsubstituted aryl, substituted or unsubstituted heteroaryl, substituted or unsubstituted cycloalkyl, substituted or unsubstituted heterocycle, or substituted or unsubstituted heterocycloalkyl; and
R is hydrogen or halogen.

10. The pharmaceutical composition of claim 9, wherein the compound represented by Formula 1 is 3-[5-(2-fluoro-phenyl)-[1,2,4]oxadiazol-3-yl]-benzoic acid.

11. The pharmaceutical composition of claim 9, wherein the neurofibromatosis is **characterized by** having of a stop codon mutation in an NF1 gene.

12. The pharmaceutical composition of claim 9, wherein the neurofibromatosis is **characterized by** increased activity of Ras proteins.

13. Use of a biomarker for predicting susceptibility to a therapeutic agent for neurofibromatosis, the biomarker comprising an agent capable of measuring an expression level of:
(1) at least one protein selected from the group consisting of a GTP-bound and activated RAS (Ras-GTP) protein and an activated p-ERK protein; or
(2) a gene encoding the at least one protein.

14. A method of predicting susceptibility of a neurofibromatosis patient to a therapeutic agent for neurofibromatosis from a biological sample obtained from an individual, comprising measuring an expression level of:
(1) at least one protein selected from the group consisting of a GTP-bound and activated RAS (Ras-GTP) protein and an activated p-ERK protein; or
(2) a gene encoding the at least one protein.

15. A method of preventing or treating neurofibromatosis, comprising administering a composition to an individual, the composition comprising, as an active ingredient, a compound represented by Formula 1 or a pharmaceutically acceptable salt thereof: 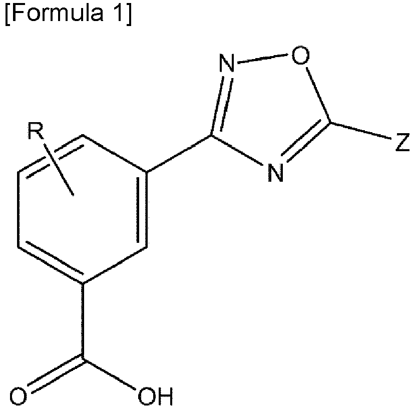
wherein, in the formula above,
Z is halogen, hydroxyl, substituted or unsubstituted alkyl, substituted or unsubstituted alkoxy, substituted or unsubstituted aryl, substituted or unsubstituted heteroaryl, substituted or unsubstituted cycloalkyl, substituted or unsubstituted heterocycle, or substituted or unsubstituted heterocycloalkyl; and
R is hydrogen or halogen.

16. Use of a composition for preparation of a therapeutic agent to be used in prevention or treatment of neurofibromatosis, the composition comprising, as an active ingredient, a compound represented by Formula 1 or a pharmaceutically acceptable salt thereof: 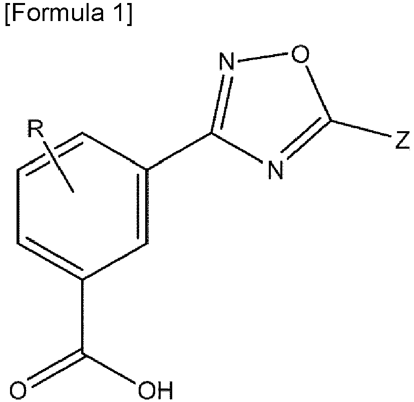
wherein, in the formula above,
Z is halogen, hydroxyl, substituted or unsubstituted alkyl, substituted or unsubstituted alkoxy, substituted or unsubstituted aryl, substituted or unsubstituted heteroaryl, substituted or unsubstituted cycloalkyl, substituted or unsubstituted heterocycle, or substituted or unsubstituted heterocycloalkyl; and
R is hydrogen or halogen.

17. A screening method by a therapeutic agent for neurofibromatosis, comprising
(a) contacting a biological sample having an NF1 gene mutation with a test substance; and
(b) measuring, in the biological sample resulting from the contacting, an expression level of: (1) at least one protein selected from the group consisting of a GTP-bound and activated RAS (Ras-GTP) protein and an activated p-ERK protein; or (2) a gene encoding the at least one protein.
